# EUROPEAN PATENT APPLICATION

(11) **EP 2 494 973 A1**
(43) Date of publication of application: **05.09.2012**
(21) Application number: 12157736.5
(22) Date of filing: 01.03.2012
(51) Int. Cl.: A61K 31/375, A61K 36/00, A61K 36/28, A61K 9/107, A61P 17/00

(54) **Topical compositions for the treatment and prevention of skin diseases, in particular radiation dermatitis caused by ionising radiation**

(30) Priority: 04.03.2011 IT MI20110339
(71) Applicant: Sinerga Group S.r.l., 20154 Milano (IT)
(72) Inventor: Guglielmini, Giancarlo, 20154 MILANO (IT); Fontana, Marco, 20154 MILANO (IT); Fontana, Alessandro, 20154 MILANO (IT)
(74) Representative: Minoja, Fabrizio

(57) **Abstract**

The present invention relates to topical compositions for the treatment and prevention of skin diseases, in particular radiation dermatitis due to ionising radiation treatments.

## Description

### Field of invention

The present invention relates to topical compositions for the treatment and prevention of skin diseases, in particular radiation dermatitis due to treatments with ionising radiation.

### Technical background

Radiotherapy treatment of tumours, and the use of technologies based on radiation for the diagnosis and treatment of tumours, constitutes an effective and extremely common therapeutic weapon against cancer. However, the side effects of radiotherapy are well known, and often severe. In particular, the damage caused by radiation to the skin and adnexa often constitutes a serious problem, which damages the quality of the life of patient or the operator.

In this context, the main skin disease is radiation dermatitis.

### Radiation dermatitis

A type of dermatitis caused by ionising radiation or radioactive substances, which affects individuals exposed to those physical agents for therapeutic purposes or in the course of their professional activity.

The biological damage caused by ionising radiation is due to:
- Ionisation of tissue fluid with the formation of toxic peroxides.
- DNA mutation.
- Permeabilisation of the cell walls caused by the release of enzymes.

In the skin, the structure most affected is the epidermis, because it consists of keratinocytes at the active replication stage. The cells of the hair, sebaceous glands, sweat glands and nails are also affected.

The skin of children and woman, which is very thin, is particularly radiosensitive.

### Acute radiation dermatitis

Caused by massive exposure to radiation in order to destroy neoplastic tissue.

Characterised by rash, swelling and stinging which regresses after 3-5 days, leaving the skin dry and flaky. In the event of major exposure, 2-3 days after the regression of the rash stage a second stage follows, with petechiae, hair loss, appearance of painful erosions, formation of scaly patches and slow repair with atrophic/pigmented sequelae, telangiectasia and xerotic skin. Exposure to sunlight worsens the lesions.

### Chronic radiation dermatitis

May arise when an acute form becomes chronic, or due to repeated absorption of small doses of radiation.

A typical skin disease affecting radiologists and surgeons who operate with the aid of radiography. The hairs on the back of the hands thin to the point of alopecia, than the nailplate thins, with the formation of longitudinal melanin streaks. The involvement of the sebaceous glands with a reduction in the production of sebum leads to skin xerosis, while a reduction in the production of melanine involves the formation of hypochromic areas.

Alterations of the blood vessels are also present which cause ulcerations and telangiectasia. Keratosis and scleroatrophic plaques then appear, which can degenerate until epitheliomas appear.

The prophylaxis of radiation dermatitis using correct irradiation techniques and suitable screening is essential.

Once the skin disease appears, it is very important to protect the skin against lesions caused by light and heat radiation, and to avoid aggressive washing and using irritant products on the skin.

Radiotherapy-induced skin reactions (ROTG) can be classified according to the following table, as reported by A. Lanfranchini, F. Ferrari, Lucio Ascani, L. Craba, P. Campanini, B. Cotza, P. Placucci and S. Serrano, in "Le Ustioni" da Raggi X - Trattamento dell'eritema bolloso.

| Grade 0 | Grade 1 | Grade 2 | Grade 3 |
|---|---|---|---|
| No change | Slight and/or painless rash Epilation, | Sensitive and/or intense rash | Flaking |
| | | | Diffuse exudation |
| | | Flaking | |
| | Flaking | Partial exudation | Marked oedema |
| | Dryness | Moderate oedema | |

The need for specific products for the prevention and treatment of radiation dermatitis, which prevent and possibly reduce the specific symptoms, is therefore strongly felt.

### Description of the invention

It has now been discovered that a composition containing a combination of vitamin C or derivatives thereof, *Echinacea angustifolia* extract and plant extracts with a high content of triterpene saponins; a lipophilic matrix comprising lecithin, trehalose and ceramides; polyphenols, from oat extract; a sunscreen; and a film-forming polymer, prevents the appearance of radiation dermatitis and reduces the symptoms of the radiation dermatitis already in progress.

The present invention therefore relates to compositions containing as active ingredients:
a) a combination of vitamin C or derivatives thereof, *Echinacea angustifolia* extract and plant extracts with a high content of triterpene saponins;
b) a lipophilic matrix containing at least 1 to 10% by weight of lecithin, trehalose and ceramides;
c) polyphenols, active ingredients extracted from oats;
d) a sunscreen;
e) a film-forming polymer;
for the treatment and prevention of skin diseases, in particular radiation dermatitis caused by treatment with ionising radiation.

The compositions according to the invention improve flaking, telangiectasia and xerotic skin in general, exercising an anti-inflammatory and soothing effect, which can also be exploited to treat dermatosis of various origins, or used for merely cosmetic purposes.

According to a preferred aspect, the compositions according to the invention contain the combination referred to in paragraph a) in the following percentages by weight:
- vitamin C phosphate: 0.1 - 2.0%;
- *Echinacea angustifolia* extract : 0.1 - 10.0%;
- *Terminalia sericea* extract : 0.1 - 5.0%.

This combination is described in Italian patent application MI2005A000874 filed on behalf of the Applicant. The combination has the effect of improving the cell repair capacity due to the synergic action between the various components, which prevent the formation of free radicals with a marked antioxidant action that increases cell turnover at the level of redox cycles, and therefore reduces the damage caused by the release of harmful radicals and increased permeabilisation of the cell membranes.

According to a preferred aspect, the compositions according to the invention contain *Echinacea angustifolia* extract, characterised by a standardised content of Echinacoside.

According to a preferred aspect, the compositions according to the invention contain *Terminalia sericea* extract in the form of a complex with phospholipids.

According to a preferred aspect, the compositions according to the invention contain a lipophilic matrix containing at least 0.5 to 10% by weight of lecithin, trehalose and ceramides as described in patent application WO 2009/044248 filed on behalf of the applicant.

This is a microencapsulated complex consisting of a pool of functional ingredients useful for the reconstruction of the skin barrier, which provides deep hydration and natural protection for the hydrolipid skin layer, and a cementing action ("intracellular filling") which improves flaking and skin xerosis.

The polyphenols present in oat extract are substances used to treat some skin conditions such as acne, eczema and dermatitis. Due to their bioactive properties they can be used to treat sensitive skin: they reduce the irritations caused on the skin by exogenous agents, such as radiation, by acting on the release of histamine, which causes skin responses such as itching, redness and swelling.

According to a preferred aspect, the compositions according to the invention contain at least 0.1% to 3% by weight of oat polyphenols.

The compositions according to the invention contain a "physical" sunscreen which guarantees adequate protection (SPF 15) without creating further cell damage. Examples of sunscreens which can be used in the compositions according to the invention are as follows: Ethylhexyl Methoxycinnamate, Butyl Methoxydibenzoylmethane, Octocrylene and Hydroxyphenyl Propamidobenzoic Acid.

According to a preferred aspect, the sunscreen is used at a minimum concentration of between 1 and 20%.

The compositions according to the invention also contain a film-forming polymer designed to form a barrier that maintains the adherence of the active ingredients to the skin surface, and to prevent excessive evaporation of fluid from the deepest layers. Examples of a film-forming polymer which can be used in the compositions according to the invention are carrageenans extracted from ocean seaweed and polymers deriving from them.

According to a preferred aspect, the film-forming polymer is used at a concentration of between 1 and 10%.

The compositions according to the invention are formulated as a W/O emulsion, which is easy to apply, not greasy, and provides immediate relief.

The pre- and post- radiotherapy formulation will be associated with a gentle cleanser for skin suffering from skin disease.

Examples of formulations according to the invention are set out below.

| **1) W/O emulsion (INCI name)** | |
|---|---|
| Polyglyceryl-3 Sorbityl Liseedate | 2.0 - 10.0% |
| Palm Glycerides | 0.5 - 3.0% |
| Potassium Lauroyl Wheat Amino Acids, Capryloyl Glycine, Dicaprylyl Ether | 2.0 - 6.0% |
| Ethylhexyl Palmitate | 2.0 - 6.0% |
| Squalane | 1.0 - 5.0 |
| Caprylic/Capric Triglyceride | 1.0 - 5.0% |
| Olea Europaea Extract | 0.5 - 2.0% |
| Chlorphenesin | 0.20% |
| Panthenol | 2.0% |
| Glycerin, Magnesium Sulphate | 1.0 - 2.0% |
| Algae | 1.0 - 7.0% |
| Glycerin | 1.0 - 3.0% |
| Echinacea Angustifolia Extract | 0.05 - 1.0% |
| Phospholipids | 0.01 - 1% |
| Terminalia Sericea Extract | 0.05 - 1.0% |
| Sodium Ascorbyl Phosphate | 0.05 - 1.0% |
| Ceramide NS | 0.05 - 3.0% |
| Trehalose | 0.05 - 5.0% |
| Cholesterol Hydrogenated Lecithin Glycerin, Octyl Dodecanol | 0.5 - 2.0% |
| Pentylene Glycol Butylene Glycol | 0.05 - 1.0% |
| Hydroxyphenyl Propamidobenzoic Acid | 0.05 - 2.0% |
| Hexanediol, Caprylyl Glycol | 0.5 - 1.0% |
| Water | to 100 G |

| **2) O/W hydrating emulsion (INCI name)** | |
|---|---|
| Echinacea Angustifolia Extract | 0.05 - 2.0% |
| Phospholipids | 0.01 - 1.0% |
| Terminalia Sericea Extract | 0.05 - 1.0% |
| Sodium Ascorbyl Phosphate | 0.05 - 2.0% |
| Ceramide NS | 0.05 - 3.0% |
| Trehalose | 0.05 - 5.0% |
| Cholesterol Hydrogenated Lecithin Glycerin, Octyl Dodecanol | 0.5 - 2.0% |
| C12 - 20 Acid Peg-8 Ester | 5.0000% |
| Glyceryl Stearate, Peg - 90 Stearate | 5.0000% |
| Polyglyceryl-3 Beeswax | 2.0000% |
| Dimethicone | 1.0000% |
| Isopropyl Myristate | 7.5000% |
| Olea Europaea Oil Unsaponifiables | 2.0000% |
| Aqua | 73.1500% |
| Disodium Edta | 0.1000% |
| Potassium Sorbate | 0.2000% |
| Phenylpropanol, Caprylyl Glycol | 0.8000% |
| Panthenol | 2.0000 |
| Glycerin, Propylene Glycol, Sodium Pca, Glucose, Urea, Glutamic Acid, Lysine, Glycine, Allantoin, Lactic Acid | 1.0000% |
| Water | to 100 G |

| **3) O/W emulsion (INCI name)** | |
|---|---|
| Echinacea Angustifolia Extract | 0.05 - 2.0% |
| Phospholipids | 0.01 - 1.0% |
| Terminalia Sericea Extract | 0.05 - 1.0% |
| Sodium Ascorbyl Phosphate | 0.05 - 2.0% |
| Ceramide NS | 0.05 - 3.0% |
| Trehalose | 0.05 - 5.0% |
| Cholesterol Hydrogenated Lecithin Glycerin, Octyl Dodecanol | 0.5 - 2.0% |
| Glycerin | 2.0% |
| Urea | 3.0% |
| Allantoin | 0.25% |
| Carbomer | 0.40% |
| Cetyl Alcohol | 2.00% |
| Potassium Palmitoyl Hydrolyzed Oat Protein, Behenyl Alcohol, Palm Glycerides, Sodium Stearoyl, Glutamate, Sucrose Palmitate | 10.00% |
| Cetearyl Isononanoate | 1.00% |
| Caprylic/Capric Triglyceride | 1.50% |
| Dicaprylyl Ether | 4.50% |
| Olea Europaea Oil Unsaponifiables | 2.00% |
| Dimethicone | 0.50% |
| Rosmarinus Officinalis Extract | 0.05% |
| Ethylhexyl Methoxycinnamate | 5.00% |
| Butyl Methoxydibenzoylmethane | 2.00% |
| Octocrylene | 1.50% |
| Polysorbate 20 | 0.50% |
| Hydrolyzed Vegetable Protein | 1.00% |
| Sodium Hyaluronate | 0.10% |
| Tocopherol | 0.15% |
| Chlorphenesin | 0.20% |
| 1,2-Hexanediol Caprylyl Glycol | 0.80% |
| Water | to 100 G |

### Pharmacological tests

Pharmacological tests have demonstrated that the compositions according to the present invention are characterised by:
- inhibition of free radicals which is 5 times more effective than that of Vitamin C;
- activity in the microcirculation, which is reduced by up to 45%, with a significant improvement in oedema and telangiectasia;
- marked moisturising activity (+21.5%).

### Evaluation of inhibition of release of VEGF (Vascular Endothelial Growth Factor) by a composition according to the invention on human keratinocytes

The aim of this test is to establish quantitatively the effects of the compositions according to the invention on the inhibition of Vascular Endothelial Growth Factor (VEGF) on human keratinocytes.

The evaluation of inhibition of release of VEGF was performed on human keratinocytes after 24 hours' treatment with a composition according to the invention.

| | |
|---|---|
| Echinacea Angustifolia Extract | 0.05 - 2.0% |
| Phospholipids | 0.01 - 1% |
| Terminalia Sericea Extract | 0.05 - 1.0% |
| Sodium Ascorbyl Phosphate | 0.05 - 2.0% |
| Ceramide NS | 0.05 - 3.0% |
| Trehalose | 0.05 - 5.0% |
| Cholesterol Hydrogenated Lecithin Glycerin, Octyl Dodecanol | 0.5 - 2.0% |

VEGF is a potent angiogenic protein that influences vascular permeability and is constitutively produced by the keratinocytes. A reduction in the production of this growth factor in the skin cells by the substance tested is therefore an a index of the ability of the product to inhibit angiogenesis and vascularisation.

VEGF Vascular Endothelial Growth Factor is a potent angiogenic protein that influences vascular permeability and is constitutively produced by the keratinocytes. Under the impulse of toxic stimuli and stressors, such as exposure to UV or to aggressive substances (SLS), the keratinocytes increase the production and release of VEGF.

The amount of VEGF was determined by the ELISA assay. This is an enzyme immunoassay based on the use of polyclonal antibodies conjugated, through a second antibody, to peroxidases. These antibodies are directed against VEGF, and the detection system is the colorimetric type due to the presence of hydrogen peroxide and a chromogenic substrate. At the end of the treatment the cell viability was analysed to check on the absence of mortality induced by the sample.

The cell model used for the *in vitro* test is represented by stabilised human keratinocytes (HaCaT cells).

### Method VEGF assay

### VEGF was assayed by the direct ELISA method.

100 µl of culture medium was left to react for 1 h at ambient temperature with the anti-VEGF polyclonal antibody bonded on 96-well plates. The wells were then washed several times with PBS (Phosphate Buffer Solution), and the secondary biotinylated monoclonal antibody was added. After 1 hour's incubation a solution of streptavidin-HP (horseradish peroxidase) was added, and the mixture left to incubate for 30 min. at ambient temperature. After suitable washing in PBS, H2O2 and the chromogenic substrate were added, leading to the development of a coloured reaction proportional to the amount of VEGF present.

A standard experimental curve of VEGF absorbance at known concentrations allows the growth factor to be quantitated in the samples analysed.

### Treatment of the sample

The cells were seeded in 6-well plates and left to grow for 24h at 37°C and 0.5% CO2.

Fresh culture medium containing the product to be tested at the concentrations of 3 and 1 mg/ml was added on the second day. The sample was dissolved in water at the concentration of 60 mg/ml and then diluted in the culture medium.

Each sample was tested in duplicate. Untreated cells were used as negative control.

At the end of the incubation the cytotoxicity test (MTT) was performed to evaluate the cell survival rate, and samples were taken for VEGF analysis with the ELISA assay.

### Results

After 24 hours' treatment, no cell mortality was observed at the composition concentrations tested.

The composition tested proved able to inhibit VEGF release at the highest concentration tested (3 mg/ml) (34% inhibition) in human keratinocytes.

### Conclusions

The *in vitro* test on the reconstituted epidermis demonstrates that the composition according to the invention reduces the production of proinflammatory agents, by 34% compared with the untreated control, and factors that influence vascular permeability (VEGF).

## Claims

1. Compositions comprising as active ingredients:
a) a combination of vitamin C or derivatives thereof, *Echinacea angustifolia* extract and extracts of plants with a high content in triterpene saponins;
b) a lipophilic matrix comprising at least 1 to 10% by weight of lecithin, trehalose and ceramides;
c) polyphenols, active constituents extracted from oats;
d) a sunscreen;
e) a polymer film-forming agent.

2. Compositions as claimed in claim 1, comprising a combination of vitamin C or derivatives thereof, *Echinacea angustifolia* extract and extracts of plants with a high content in triterpene saponins in the following percentages by weight:
- vitamin C phosphate: 0.1 - 2.0%;
- *Echinacea angustifolia* extract: 0.1 - 10.0%;
- *Terminalia sericea* extract: 0.1 - 5.0%.

3. Compositions as claimed in claim 1, wherein the *Echinacea angustifolia* extract is **characterized by** a standardized Echinacoside content.

4. Compositions as claimed in claim 1, wherein the *Terminalia sericea* extract is present as a complex with phospholipids.

5. Compositions as claimed in claim 1, wherein the lipophilic matrix is present in a concentration of 0.5% to 10%.

6. Compositions as claimed in claim 1, wherein the polyphenols from oat extract are present in a concentration of 0.1% to 3.

7. Compositions as claimed in claim 1, wherein the sunscreen is selected from ethylhexyl methoxycinnamate, butyl methoxydibenzoylmethane, octocrylene and hydroxyphenyl propamidobenzoic acid.

8. Compositions as claimed in claim 1, wherein the sunscreen is present in a minimum concentration of 1%.

9. Compositions as claimed in claim 1, wherein the polymer film-forming agent is selected from carrageenans extracted from ocean seaweeds and polymers derived therefrom.

10. Compositions as claimed in claim 1, wherein the polymer film-forming agent is present in a concentration of 1 to 10%.

11. Compositions according to the above claims, for the treatment and prevention of dermopathies.

12. Compositions as claimed in claim 11, wherein the dermopathies are radiation dermatitis due to treatments with ionizing radiation.

13. Compositions as claimed in claim 11, wherein the dermopathies are caused by localized inflammation of human body cavities, such as the auricular, oral, anal and vaginal cavities.

14. Compositions according to the above claims, in the form of W/O emulsion, O/W fluid emulsions, O/W thick emulsions, moisturizing creams and/or nutrients.
